# FASCICULE DE BREVET EUROPEEN

(11) **EP 1 086 666 B1**
(45) Date de publication et mention de la délivrance du brevet: **10.11.2004**
(21) Numéro de dépôt: 00420190.1
(22) Date de dépôt: 12.09.2000
(51) Int. Cl.: A61F 2/34

(54) **Implant cotyloidien**
Hüftgelenkpfannenimplantat
Cotyloidal implant

(30) Priorité: 21.09.1999 FR 9911913
(43) Date de publication de la demande: 28.03.2001
(73) Titulaire: Chambaud, Denis, 42153 Riorges (FR); Lucet, Alain, 21121 Fontaine (FR); Pere, Christian, 37260 Artannes sur Indre (FR); Moretton, Jean Claude, 88000 Epinal (FR); Capon, Didier, 44880 Sautron (FR); Grobost, Jérome, 72000 Le Mans (FR)
(72) Inventeur: Chambaud, Denis, 42153 Riorges (FR); Lucet, Alain, 21121 Fontaine (FR); Pere, Christian, 37260 Artannes sur Indre (FR); Moretton, Jean Claude, 88000 Epinal (FR); Capon, Didier, 44880 Sautron (FR); Grobost, Jérome, 72000 Le Mans (FR)
(74) Mandataire: Thivillier, Patrick

(56) Documents cités:
- EP-A- 0 308 297
- EP-A- 0 663 193
- EP-A- 0 743 050
- EP-A- 0 775 475
- EP-A- 0 826 347
- EP-A- 0 841 041
- DE-A- 3 630 276
- DE-U- 29 516 473
- FR-A- 2 641 461
- FR-A- 2 682 588
- FR-A- 2 703 240
- FR-A- 2 715 828
- FR-A- 2 719 465
- FR-A- 2 720 625

## Description

L'invention se rattache au secteur technique des implants orthopédiques pour l'articulation de la hanche.

D'une manière parfaitement connue, on rappelle qu'un implant cotyloïdien comprend, pour l'essentiel, une cupule métallique de forme générale hémisphérique destinée à recevoir un insert ou noyau coopérant avec une tête fémorale. La cupule métallique est impactée dans la cavité cotyloïdienne de l'os iliaque en y étant maintenue par différents moyens, en combinaison ou non avec du ciment. Notamment, la fixation de la cupule peut être assurée par un effet connu sous le nom de "PRESS-FIT". Le noyau peut être monté dans la cupule, d'une manière fixe ou avec capacité de déplacement multidirectionnel. L'invention concerne plus particulièrement un implant cotyloïdien dans lequel le noyau est monté dans la cavité interne concave de la cupule sans aucune possibilité de déplacement, c'est-à-dire d'une manière fixe.

En ce qui concerne le noyau, ce dernier peut être en matière plastique, notamment polyéthylène, en céramique ou encore en métal.

L'intérêt d'utiliser un noyau céramique ou métallique se trouve dans la réduction du couple de frottement articulaire en raison de la dureté de la surface et par conséquent de l'usure sous l'effet du déplacement multidirectionnel de la tête fémorale. Par contre, l'utilisation d'un noyau en céramique notamment est soumise à certaines contraintes :
- compte tenu de la rigidité du matériau, la cavité interne de la cupule doit rester parfaitement rigide et indéformable ;
- compte tenu de la fragilité aux chocs du matériau, tout effet de contact du col prothétique avec le bord externe de l'insert est proscrit ; il n'y a donc pas de possibilité de réalisation de prolongement de l'insert hors de la cupule pour un effet anti-luxation ;
- compte tenu de la rigidité du système noyau céramique/cupule, les cas d'indications d'un tel système sont limités aux cavités acétabulaires de diamètres faibles ou moyens et de qualité osseuse suffisante.

A l'inverse, un noyau en polyéthylène offre une plus grande souplesse d'utilisation de par son caractère amortissant et la possibilité de lui intégrer une parade anti-luxation mais présente des risques de fluage et d'usure importants sous charge et en mouvement.

A partir de cet état de la technique, les cupules actuellement utilisées prennent le parti
- soit d'être parfaitement rigides et de proposer à volonté, soit un insert en céramique, soit un insert en polyéthylène ;
- soit d'être élastiques et de proposer exclusivement un insert en polyéthylène.

Le problème que se propose de résoudre l'invention est de pouvoir adapter au choix un insert polyéthylène ou céramique dans une cupule présentant une élasticité propre à autoriser son implantation en PRESS-FIT et à se conformer au plus près aux déformations acétabulaires sous charge.

Une telle invention permet d'optimiser simultanément la fixation de la cupule à l'os et le frottement de la tête dans l'insert.

Le brevet FR2.703.240 décrit une cupule comportant des moyens pour que sa face externe se déforme élastiquement, tandis que sa partie interne reste rigide et indéformable. Les moyens de déformation décrits dans ce brevet sont constitués par des fentes pratiquées dans l'épaisseur de la cupule. Toutefois, de par la conception de ces fentes, la déformation élastique n'apparaît pas suffisante pour autoriser un PRESS-FIT et une déformation adaptée à la charge dans tous les cas osseux standards.

L'invention s'est fixée pour but de remédier à ces inconvénients, de manière simple, sûre, efficace et rationnelle.

Le problème que se propose de résoudre l'invention est de pouvoir équiper à volonté une cupule déformable élastiquement soit d'un noyau en céramique, soit d'un noyau en polyéthylène.

Pour résoudre un tel problème, il a été conçu et mis au point un implant cotyloïdien comprenant une cupule métallique de forme générale hémisphérique destinée à recevoir un insert ou noyau coopérant avec une tête fémorale, ladite cupule présentant des moyens pour que sa surface externe se déforme par élasticité et que sa surface interne concave recevant le noyau reste rigide et indéformable, la surface interne de la cupule présentant des agencements de fixation aptes à coopérer à volonté avec des agencements complémentaires soit d'un noyau en matière plastique notamment en polyéthylène, soit d'un noyau en matière céramique.

Pour résoudre le problème posé d'assurer la fixation soit d'un noyau en polyéthylène, soit d'un noyau en céramique, dans une même cupule, les agencements de fixation de la surface interne de la cupule, sont constitués, d'une part, par une série de portées tronconiques formées à partir de son ouverture, la première de ces portées coopérant avec une forme complémentaire du noyau en céramique pour créer un effet de coincement et, d'autre part, par une gorge formée à l'intersection desdites portées et apte à coopérer avec une nervure circulaire que présente la surface externe du noyau polyéthylène, pour assurer sa fixation par un effet de clipage.

Pour résoudre le problème posé d'assurer le blocage en rotation du noyau en polyéthylène, la surface intérieure de la cupule présente sous la portée tronconique des ergots métalliques aptes à pénétrer la surface polyéthylène au moment de l'impaction du noyau.

Pour résoudre le problème posé d'assurer une déformation de la cupule, aussi bien au moment de l'impaction que pendant la marche, les moyens de déformation élastique de la surface externe de la cupule, sont constitués par au moins une fente verticale débouchante au niveau de ladite surface externe, ladite fente étant en communication avec une fente formée dans l'épaisseur de la cupule, de part et d'autre et symétriquement.

Pour résoudre le problème posé d'améliorer l'élasticité et par conséquent la déformation de la surface externe de la cupule, de part et d'autre de la fente formée dans l'épaisseur de la cupule, sont formées deux autres fentes dont l'une des extrémités située du côté de ladite fente centrale est en communication avec une fente verticale débouchante au niveau de la surface externe de ladite cupule.

Pour résoudre le problème posé d'améliorer la stabilité de la cupule, dans la cavité cotyloïdienne, la surface externe de la cupule présente des ailettes verticales de stabilité formées en retrait de son bord supérieur et sur une hauteur limitée. Cette stabilité est améliorée pour un fond aplati de la cupule.

Dans une forme de réalisation, le fond de la cupule présente des trous débouchants pour l'engagement de vis de fixation dans la cavité cotyloïdienne de l'os iliaque.

L'invention est exposée ci-après plus en détail à l'aide des figures des dessins annexés dans lesquels :

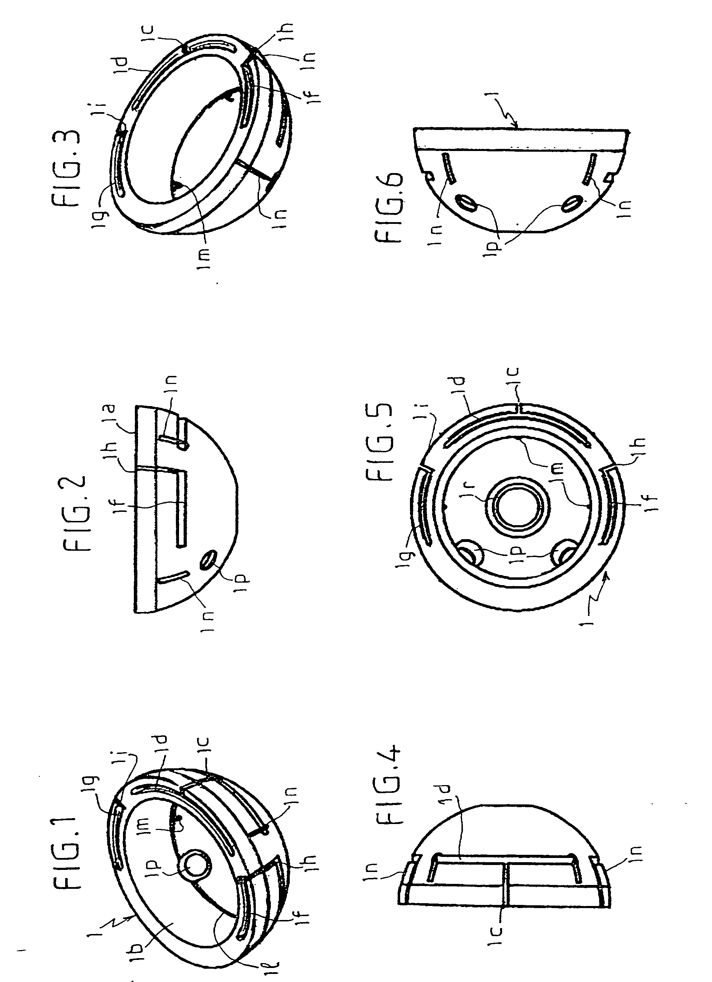

- les figures 1 à 6 sont des vues de face, de côté et en perspective de la cupule métallique selon les caractéristiques de l'invention ;

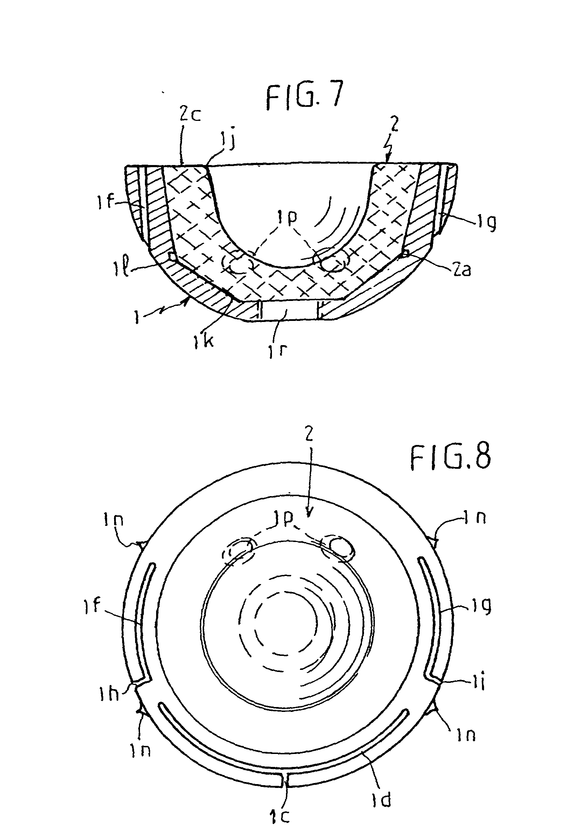

- la figure 7 est une vue en coupe longitudinale de la cupule après fixation du noyau en céramique ;
- la figure 8 est une vue en plan correspondant à la figure 7 ;

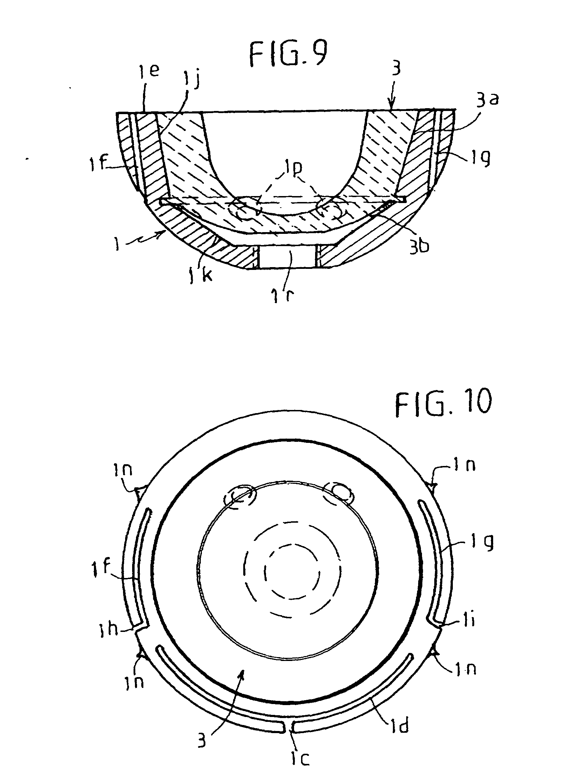

- la figure 9 est une vue en coupe longitudinale de la cupule après fixation du noyau en polyéthylène ;
- la figure 10 est une vue en plan correspondant à la figure 9.

Selon l'invention, l'implant cotyloïdien comprend une cupule (1) présentant des agencements pour recevoir à volonté soit un noyau en matière plastique, notamment en polyéthylène (2), soit un noyau en matière céramique (3). La cupule (1) est métallique et de forme générale hémisphérique en étant conformée pour être fixée dans le fond de la cavité cotyloïdienne notamment par effet de PRESS-FIT. La cupule (1) présente en outre des moyens pour que la surface externe (1a) se déforme par élasticité, et que sa surface interne concave (1b) reste rigide et indéformable.

Dans ce but, la surface externe (1a) de la cupule (1) présente au moins une fente verticale débouchante (1c) en communication avec une fente (1d) formée dans l'épaisseur de la cupule (1) à partir de son bord supérieur circulaire (1e). La fente (1d) est établie de part et d'autre de la fente verticale (1c) et symétriquement à cette dernière. Après impaction de la cupule dans la cavité osseuse, la fente centrale (1d) coopère avec les cornes cotyloïdiennes. Pour améliorer l'élasticité, deux autres fentes (1f et 1g) sont formées de part et d'autre de la fente centrale (1d). Les fentes (1f et 1g) sont formées, de la même façon que la fente centrale (1d), dans l'épaisseur de la cupule (1) et sont en communication au niveau de l'une de leurs extrémités seulement, avec une fente (1h et 1i) formée verticalement.

Selon une caractéristique importante de l'invention, la surface interne (1b) de la cupule présente des agencements de fixation aptes à coopérer à volonté avec des agencements complémentaires soit du noyau en polyéthylène (2), soit du noyau en céramique (3). A cet égard, la surface interne de la cupule présente, à partir du bord (1e) une séries de portées circulaires tronconiques (1j et 1k). La portée tronconique (1j) la plus proche de l'ouverture est destinée à coopérer avec une portée circulaire tronconique mâle complémentaire (3a) que présente le noyau en céramique (3). La conicité de la portée mâle (3a) est voisine de la conicité de la portée femelle (1g) pour assurer une fixation du noyau dans la cavité (1b) par effet de coincement. La portée circulaire tronconique (3a) du noyau (3) est prolongée par une portion sphérique (3b) qui n'est pas en contact avec la portée circulaire tronconique (1k) de la cupule.

La surface interne (1b) de la cupule (1) présente une gorge (1l) formée à l'intersection des portées circulaires tronconiques (1j et 1k). Cette gorge circulaire (1l) est formée parallèlement au plan équatorial délimité par le bord supérieur (1e). Cette gorge (1l) reçoit une nervure circulaire (2a) que présente la surface externe du noyau en polyéthylène (2), afin d'assurer sa fixation par un effet de clipage. Pour assurer le blocage en rotation du noyau en polyéthylène (2), la cavité interne (1b) de la cupule (1), présente trois ergots métalliques (1m) destinés à pénétrer la surface polyéthylène du noyau (2) au moment de son impaction.

Le noyau (2) présente des portées circulaires tronconiques coopérant avec les portées circulaires tronconiques de la cupule.

Selon une autre caractéristique, la surface externe de la cupule (1) présente des ailettes verticales (1n) formées en retrait de son bord supérieur (1e) et sur une hauteur limitée. Ces ailettes (1n) ont une section transversale sensiblement triangulaire. Avantageusement, la cupule présente quatre ailettes décalées angulairement de 90°.

Pour améliorer la fixation de la cupule dans la cavité cotyloïdienne, le fond de cette dernière peut présenter des trous débouchants (1p) pour l'engagement de vis de tous types connus et appropriés. De même, le fond de la cavité cotyloïdienne peut présenter un trou (1r), notamment pour le montage d'un appareil impacteur.

De même, la cupule (1) est aplatie au niveau du pôle pour assurer son contact à l'os sur les parois acétabulaires préférentiellement à un contact en fond de cavité.

Les avantages ressortent bien de la description, en particulier on souligne et on rappelle :
- la possibilité d'utiliser, à partir d'une même cupule, soit un noyau en matière plastique du type polyéthylène, soit un noyau en matière rigide, notamment en céramique ;
- la déformation élastique de la surface externe de la cupule au moment de son impaction et sous charge, sans modifier la surface interne qui demeure rigide et indéformable.

## Revendications

1. Implant cotyloïdien comprenant une cupule métallique (1) de forme générale hémisphérique destinée à recevoir un noyau (2 - 3) coopérant avec une tête fémorale, ou la cupule (1) présente des moyens pour que sa surface externe se déforme par élasticité et que sa surface interne concave recevant le noyau reste rigide et indéformable ; et **caractérisé en ce que**,
- la surface interne (1b) de la cupule présente des agencements de fixation aptes à coopérer à volonté avec des agencements complémentaires soit d'un noyau en matière plastique notamment en polyéthylène (2), soit d'un noyau en matière céramique (3); et
- la cupule metallique est destinée à être impactée par effet de « press-fit ».

2. Implant selon la revendication 1, **caractérisé en ce que** les agencements de fixation de la surface interne (1b) de la cupule (1), sont constitués, d'une part, par une séries de portées circulaires tronconiques (1j et 1k) formées à partir de son ouverture, la première de ces portées (1j) coopérant avec une forme complémentaire du noyau en céramique (3) pour créer un effet de coincement et, d'autre part, par une gorge (1l) formée à l'intersection desdites portées (1j et 1k) et apte à coopérer avec une nervure circulaire (2a) que présente la surface externe du noyau polyéthylène (2).

3. Implant selon la revendication 1, **caractérisé en ce que** la surface interne (1e) de la cupule présente, sous la portée tronconique, des ergots métalliques aptes à pénétrer la surface polyéthylène au moment de l'impaction du noyau (2).

4. Implant selon la revendication 1, **caractérisé en ce que** les moyens de déformation élastique de la surface externe (1a) de la cupule (1), sont constitués par au moins une fente verticale débouchante (1e) au niveau de ladite surface externe, ladite fente (1c) étant en communication avec une fente (1d) formée dans l'épaisseur de la cupule de part et d'autre et symétriquement.

5. Implant selon la revendication 4, **caractérisé en ce que**, de part et d'autre de la fente (1d) formée dans l'épaisseur de la cupule, sont formées deux autres fentes (1f et 1g) dont l'une des extrémités située du côté de ladite fente (1d) est en communication avec une fente verticale (1h - 1i) débouchante au niveau de la surface externe de ladite cupule (1).

6. Implant selon la revendication 1, **caractérisé en ce que** la surface externe (1b) de la cupule (1) présente des ailettes verticales de stabilité formées en retrait de son bord supérieur et sur une hauteur limitée.

7. Implant selon la revendication 1, **caractérisé en ce que** le fond de la cupule présente des trous débouchants (1p) pour l'engagement de vis de fixation dans la cavité cotyloïdienne de l'os iliaque.

8. Implant selon la revendication 7, **caractérisé en ce que** le fond de la cupule (1) est aplatie.

## Claims

1. Acetabular implant comprising a metal cup (1) of general hemispherical shape intended to receive a core (2-3) cooperating with a femoral head, the cup (1) having means such that its outer surface deforms by elasticity and its concave inner surface receiving the core remains rigid and non-deformable; and **characterized in that**
- the inner surface (1b) of the cup has fixing arrangements which are able to cooperate with complementary arrangements either on a core made of plastic material, in particular polyethylene (2), or on a core made of ceramic material (3); and
- the metal cup is intended to be impacted by a press-fit effect.

2. Implant according to Claim 1, **characterized in that** the fixing arrangements on the inner surface (1b) of the cup (1) are formed, on the one hand, by a series of truncated circular spans (1j and 1k) formed starting from its opening, the first of these spans (1j) cooperating with a complementary shape of the ceramic core (3) to create a wedging effect, and, on the other hand, by a groove (11) formed at the intersection of said spans (1j and 1k) and able to cooperate with a circular rib (2a) arranged on the outer surface of the polyethylene core (2).

3. Implant according to Claim 1, **characterized in that** the inner surface (1e) of the cup has, under the truncated span, metal studs which are able to penetrate the polyethylene surface at the moment of impaction of the core (2).

4. Implant according to Claim 1, **characterized in that** the means of elastic deformation of the outer surface (1a) of the cup (1) are formed by at least one open vertical slot (1c) on said outer surface, said slot (1c) communicating with a slot (1d) formed in the thickness of the cup on each side and symmetrically.

5. Implant according to Claim 4, **characterized in that**, on either side of the slot (1d) formed in the thickness of the cup, two other slots (1f and 1g) are formed, of which one end situated towards said slot (1d) is in communication with an open vertical slot (1h - 1i) on the outer surface of said cup (1).

6. Implant according to Claim 1, **characterized in that** the outer surface (1b) of the cup (1) has vertical fins for stability which are formed set back from its upper edge and over a limited height.

7. Implant according to Claim 1, **characterized in that** the bottom of the cup has through-holes (1p) for engagement of fixing screws in the acetabular cavity of the iliac bone.

8. Implant according to Claim 7, **characterized in that** the bottom of the cup (1) is flattened.

## Patentansprüche

1. Hüftgelenkpfannenimplantat mit einer allgemein halbkugelförmigen Metallschale (1), die einen Einsatz (2 - 3 ) aufnimmt, der mit einem Hüftkopf zusammenwirkt, die Schale (1) Elemente besitzt, damit sich ihre Außenfläche elastisch verformt und ihre konkave Innenfläche, die den Einsatz aufnimmt, starr und formstabil ist, **dadurch gekennzeichnet, dass** die Innenfläche (1b) der Schale Befestigungsanordnungen besitzt die bei Bedarf mit zusätzlichen Anordnungen eines Kunststoffeinsatzes, insbesondere aus Polyäthylen (2), oder eines Keramikeinsatzes (3) zusammenwirken können und die Metallschale durch "Press-Fit"-Wirkung befestigt wird.

2. Implantat nach Anspruch 1, **dadurch gekennzeichnet, dass** die Anordnungen zur Befestigung der Innenfläche (1b) der Schale (1) einerseits aus einer Reihe kegelstumpfartiger, kreisförmiger Auflageflächen (1j - 1k) bestehen, die an seiner Öffnung gebildet werden, wobei die erste dieser Auflageflächen (1j) mit einer zusätzlichen Form des Keramikeinsatzes (3) zusammenwirkt, so dass ein Einklemmefiekt erreicht wird, und andererseits aus einer Kehle (11) besteht, die am Schnittpunkt der Auflageflächen (1j und 1k) gebildet wird, und mit einer kreisförmigen Rippe (2a) zusammenwirken kann, welche die Außenfläche des Einsatzes aus Polyäthylen (2) darstellt.

3. Implantat nach Anspruch 1, **dadurch gekennzeichnet, dass** die Innenfläche (1e) der Schale unter der kegelstumpfartigen Auflagefläche Metallvorsprünge besitzt, welche die Polyäthyleufläche beim Auftreffen des Einsatzes (2) durchdringen können.

4. Implantat nach Anspruch 1, **dadurch gekennzeichnet, dass** die Elemente elastischer Verformung der Außenfläche (1a) der Schale (1) aus mindestens einem vertikalen Schlitz (1c) bestehen, der im Bereich der Außenfläche offen ist, wobei der Schlitz (1c) mit einem Schlitz (1d) in Verbindung steht, der zu beiden Seiten der Schale symmetrisch gebildet wird.

5. Implantat nach Anspruch 4, **dadurch gekennzeichnet, dass** zu beiden Seiten des Schlitzes (1d) in der Schale zwei weitere Schlitze (1f und 1g) gebildet werden, wobei jeweils ein Ende an der Seite des Schlitzes (1d) mit einem vertikalen Schlitz (1h - li) in Verbindung steht, der im Bereich der Außenfläche der Schale (1) offen ist.

6. Implantat nach Anspruch 1, **dadurch gekennzeichnet, dass** die Außenfläche (1b) der Schale (1) vertikale Stabilisierungsrippen besitzt, die zurückgesetzt zum oberen Rand in begrenzter Höhe angeordnet sind.

7. Implantat nach Anspruch 1, **dadurch gekennzeichnet, dass** sich im Boden der Schale zwei Öffnungen (1p) für den Eingriff von Befestigungsschrauben in die Hüftgelenkpfannenvertiefung des Hüftknochens befinden.

8. Implantat nach Anspruch 7, **dadurch gekennzeichnet, dass** der Boden der Schale (1) flach ist
